# EUROPÄISCHE PATENTSCHRIFT

(11) **EP 1 048 882 B1**
(45) Veröffentlichungstag und Bekanntmachung des Hinweises auf die Patenterteilung: **30.11.2005**
(21) Anmeldenummer: 99108143.1
(22) Anmeldetag: 26.04.1999
(51) Int. Cl.: F16L 19/02, A61M 39/10

(54) **Rohrverbindung**
Pipe connection
Raccord de tuyaux

(43) Veröffentlichungstag der Anmeldung: 02.11.2000
(73) Patentinhaber: Möller Feinmechanik GmbH & Co. KG, 36043 Fulda (DE)
(72) Erfinder: Reinhardt, Werner, 36137 Grossenlüder (DE); Frank, Michael, 36137 Grossenlüder (DE); Herget, Bernhard, 36115 Ehrenberg 3 (DE)
(74) Vertreter: Hasler, Erich

(56) Entgegenhaltungen:
- EP-A- 0 611 174
- DE-A- 19 635 090

## Beschreibung

Die Erfindung betrifft eine Rohrverbindung zur Verbindung der Rohrenden von zwei Rohren, mit Dichtmitteln und einer Spann- oder Zusammenziehvorrichtung zum Zusammenziehen der Rohrenden.

Insbesondere in der Medizin und in der Analysetechnik ist von Bedeutung, dass Transportleitungen oder rohre, in welchen z.B. Analysemedien in Gas- oder flüssiger Form transportiert werden, möglichst keine Totvolumina aufweisen. Totvolumina sind von Spülmedien nicht oder nur schlecht zu erreichen, sodass es in einer Leitung, in welcher ein Totvolumen oder eine Totecke vorhanden ist, zu Verschleppungen der transportierten Medien kommen kann. Kommt z.B. in einer Analyseprobe eine bestimmte Komponente in einer höheren Konzentration vor, so wird - wenn der Transportweg der Analyseprobe zum Messgerät ein Totvolumen aufweist - möglicherweise dieselbe Komponente auch in der nachfolgend gemessenen Probe festgestellt, obwohl die nachfolgende Probe in Wirklichkeit die fragliche Komponente nicht aufweist und die Leitung zwischendurch gründlich gespült wurde.

Besonders kritisch bezüglich Totvolumina sind erfahrungsgemäss Rohrverbindungen, da bekannte Rohrbindungssysteme sehr oft keinen totvolumen- oder totwassereckenfreien Übergang von einem Rohr zum nächsten ermöglichen. In der Regel bleibt irgendwo ein Totvolumen zwischen einer Dichtung und den Rohrenden übrig, welches in der Analytik zu Fehlmessungen führen kann. Totvolumina können sich z.B. dann ergeben, wenn die Dichtung nicht richtig sitzt oder die Rohrenden und die Dichtungen nicht genau zusammengefügt wurden. In der Praxis besteht deshalb ein grosses Bedürfnis für totvolumenminimierte Rohrverbindungen, insbesondere bei Feinrohren mit einem Aussendurchmesser von weniger als 2 bis 3 Millimetern.
Aus der EP-A-0 611 174 ist eine Vorrichtung gemäss dem Oberbegriff des Anspruchs 1 bekannt. Diese dient zur Verbindung eines ersten Rohrendes aus einem nichtmetallischen Material mit einem zweiten Rohrende aus einem metallischen Material. Eine zylindrische Muffe ist auf das erste, nicht-metallische Rohrende aufgeklebt. Die Muffe besitzt einen ringförmigen Kragen, welcher fluchtend mit der Innenfläche des Rohres ist. Das zweite, metallische Rohrende ist in einen Spannring eingeschraubt. In der Stirnfläche des Spannrings ist eine umlaufende Nut vorgesehen, in welcher ein Dichtring eingelegt ist. Der Spannring und eine Überwurfsmutter sorgen zusammen für eine Zentrierung der beiden Rohrenden.
Aus der DE 196 35 090 ist eine Rohrverbindung für grosse Rohrdurchmesser bekannt, welche vor allem als Hochdruckverschraubung zum Einsatz unter Tage eingesetzt wird. Die Rohrverbindung besteht aus einem Steckteil und einem Muffenteil, welche formschlüssig zusammenwirken. Das Muffenteil und das Steckteil sind an den Rohrenden zweier Rohre angeschweisst. Die Dichtung erfolgt über einen Dichtring, welcher in einer Ringnut auf der Aussenseite des Steckteils eingelegt ist.

Es ist deshalb Aufgabe der vorliegenden Erfindung eine Feinrohrverbindung bereitzustellen, welche möglichst kein Totvolumen aufweist. Die Rohrverbindung sollte von Fach- wie Hilfskräften schnell und ohne grosse Kontroll- und Justierarbeiten eingesetzt werden können und dabei einen praktisch totvolumenfreien Übergang ermöglichen.

Erfindungsgemäss wird dies durch eine Rohrverbindung gemäss dem Anspruch 1 gelöst. Diese Rohrverbindung dient zur Verbindung der Rohrenden von zwei Rohren, weist Dichtmitteln und eine Spann- oder Zusammenziehvonichtung zum Zusammenziehen der Rohrenden auf. Bei dieser Rohrverbindung ist auf dem ersten Rohrende ein erstes Kopfteil aus Kunststoff ausgebildet. An diesem Kopfteil ist ein Flansch ausgebildet, welcher einen mit dem Innendurchmesser des ersten Rohrendes fluchtenden Durchgang besitzt. Dieser Flansch wirkt einerseits mit der Stirnseite des ersten Rohrendes zusammen und besitzt andererseits eine sich quer zur Längsachse der Rohre erstreckende Dichtfläche. Auf dem zweiten Rohrende ist ein zweites Kopfteil ausgebildet. Es sind ferner miteinander zusammenwirkende Führungsmittel vorhanden, welche sicherstellen , dass bei zusammengezogenen Rohrenden der Durchgang des Flansches und der Innendurchmesser des zweiten Rohrendes miteinander fluchten. Erfindungsgemäss sind bei einer solchen Rohrverbindung die Rohre metallische Feinrohre und die Kopfteile auf die Rohrenden aufgespritzte Kunststoff-Umspritzungen. Die miteinander zusammenwirkenden Führungsmittel sind an den Kopfteilen ausgebildet und die beiden Kopfteile zusammensteckbar. Der am ersten Kopfteil ausgebildete Flansch ist eine Dichtung, deren Dichtfläche bei zusammengezogenen Rohrenden spaltfrei am zweiten Rohrende und/oder am zweiten Kopfteil anliegt. Eine solche Rohrverbindung hat den Vorteil, dass diese wegen der vorhandenen Führungsmittel mühelos von Fach- und Hilfspersonal montiert werden kann. Durch das Vorsehen einer vorzugsweise elastischen oder unter Druck verformbaren Dichtung zwischen den Rohrenden kann der Übergang zwischen zwei Rohren praktisch nahtlos sein.

Obwohl in bestimmten Fällen eine einzige Dichtung ausreicht, sieht eine bevorzugte Ausführungsform der Rohrverbindung vor, dass sowohl am ersten Kopfteil als auch am zweiten Kopfteil eine Dichtung angeformt ist. Von Bedeutung ist, dass die Dichtung mit der Innenwandung des aufzunehmenden Rohres möglichst genau fluchtet.

Die Führungsmittel können z.B. als männliches und als weibliches Teil ausgebildet sein, welche zusammensteckbar sind. Vorteilhaft wirken die Führungsmittel der beiden Kopfteile formschlüssig miteinander zusammen. Die Führungsmittel können sich über den ganzen Umfang der Kopfteile erstrecken und können z.B. als zylindrischer Stutzen oder Zapfen und als zylindrische Aussparung ausgebildet sein.

Gemäss einer bevorzugten Ausführungsform ist der Zapfen leicht konisch, Dies hat den Vorteil, dass die Justierung besonders gut gelingt.

Die Realisierung der Kopfteile als Umspritzung hat den grossen Vorteil, dass zwischen Rohrwand und Dichtung ein nahtloser Übergang besteht.

Vorteilhaft sind in Abstand zum Rohrende Befestigungselemente zur wenigstens axialen Fixierung der Kopfteile vorgesehen. Diese können beispielsweise als ein an das Rohr geschweisster Ring oder als eine Bördelung des Rohrmantels realisiert sein. Die Befestigungselemente können der Befestigung der Kopfteile am Rohr und als verbreiterte Dichtungsabstützung dienen. Zweckmässigerweise sind die Befestigungselemente mit Aussparungen versehen. Diese Aussparungen verbessern die Haftung der Kopfteile am Rohr. Sie haben den Vorteil, dass der flüssige Kunststoff beim Spritzvorgang durch die Aussparungen fliessen kann und somit überall hinkommt. Die Befestigungselemente sind vorteilhaft in einem Abstand von < 3 mm, vorzugsweise < 1 mm und ganz besonders bevorzugt < 0.5 mm zum Rohrende angeordnet.

Die Dichtmittel können das erste Kopfteil und das zweite Kopfteil umfassen, wobei das erste Kopfteil am ersten Rohrende und das zweite Kopfteil am zweiten Rohrende - jeweils als Umspritzung der Rohrenden - angeordnet ist. An den Kopfteilen ist eine Dichtung angeformt, welche die Stirnseiten der Rohrenden bedeckt und fluchtend mit der Innenwandung der Rohre ist. Die beiden Kopfteile weisen miteinander zusammenwirkende Führungsmittel auf und sind zusammensteckbar, sodass die Rohrquerschnitte miteinander fluchten. Es besitzen die Dichtungen der Kopfteile jeweils eine quer zur Längsachse der Rohre sich erstreckende Dichtfläche, welche an den Stirnseiten der Rohrenden, bzw. aneinander anliegen. Damit kann eine dichte Verbindung zwischen zwei Rohren realisiert werden.

Als Spannvorrichtung zum Zusammenziehen der Rohre kann z.B. ein Schraube und eine entsprechende Überwurfmutter eingesetzt werden. Es sind aber auch andere Spannmittel anwendbar, die ähnlich wirken.

Es zeigt
- Figur 1:: eine erste Ausführungsform einer erfindungsgemässen Rohrverbindung im Längsschnitt;
- Figur 2:: ein Rohrendstück mit einem aufgespritzten Kunststoff-Kopfteil mit Dichtung und einer Überwurfmutter mit leitender Verbindung zum Rohr im Schnitt;
- Figur 3:: eine zweite Ausführungsform einer erfindungsgemässen Rohrverbindung mit nur einer Dichtung zwischen den Rohrenden, ebenfalls im Längsschnitt.

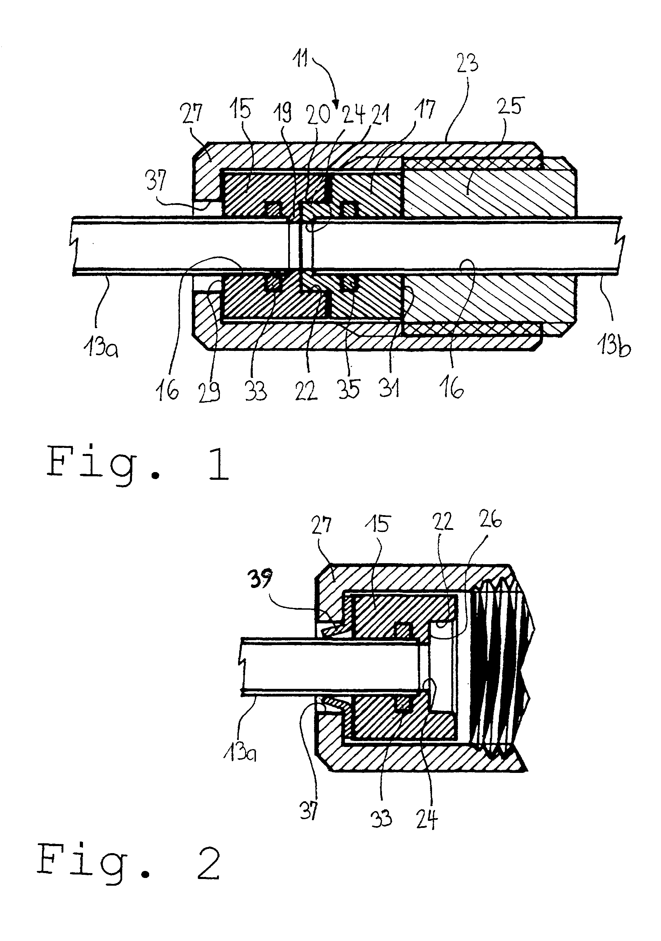

Das in Figur 1 dargestellte erste Ausführungsbeispiel zeigt eine mittels einer Rohrverbindung 11 bewirkte Verbindung zweier Rohre 13a, 13b. Die Rohrverbindung 11 besteht aus zwei Kopfteilen 15,17 in Form von Ringen mit jeweils daran angeformten Dichtungen 19,21 und einer Klemm- oder Spannvorrichtung 23.

Die Kopfteile 15,17 besitzen die Gestalt von Ringen, welche auf die Rohrenden aufgesetzt oder mit diesen unlösbar verbunden sind. Sie besitzen jeweils ein Loch 16 zur Aufnahme eines Rohrendes 13a resp. 13b. Von Bedeutung ist nun, dass die Kopfteile 15,17 miteinander korrespondierende Führungsmittel aufweisen, die bewirken, dass die Löcher 16 der Kopfteile 15,17 exakt miteinander fluchten, wenn die Kopfteile zusammengesteckt sind. Zu diesem Zweck hat das Kopfteil 17 einen Zapfen 20 und das Kopfteil 15 eine Aussparung 22, welche formschlüssig miteinander zusammenwirken.

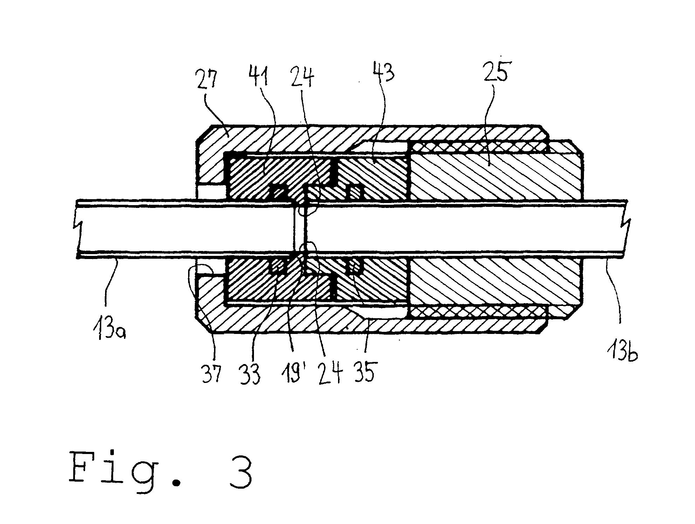

Die an den Kopfteilen 15,17 angeformten Dichtungen 19,21 sind als Flansche ausgebildet, welche dem Innendurchmesser der zu verbindenden Rohre 13a,13b entsprechende Durchgänge 24 besitzen. Der Übergang von der verbindungsseitigen Dichtfläche 26 (Figur 2) zur Innenkante des Durchgangs 24 ist kantig, sodass die Verbindung zwischen den beiden Dichtungen bei zusammengeschraubter Rohrverbindung im wesentlichen spalt- und damit totvolumenfrei ist. Die Stärke der Dichtungen 19,21 im Bereich der Rohrenden beträgt bei einem Rohr mit Aussendurchmesser 2 mm vorzugsweise ca. 0.5 mm.

Die aus einer Schraube 25 und einer Überwurfmutter 27 bestehende Spannvorrichtung 23 greift an den verbindungsfernen Stirnseiten 29 resp. 31 der Kopfteile 19,21 an und presst diese zusammen. Die Kopfteile 19,21 umschliessen Befestigungselemente 33,35 welche mit den Rohren 13a resp. 13b fest verbunden sind. Abstand dazu angeordnet. Im Falle des Kopfteils 17 ist das Befestigungselement 35 wegen des Stutzens 20 etwas weiter vom Rohrende angeordnet als beim Kopfteil 15. Die Befestigungselemente 33,35 bewirken eine gute Verbindung der Kopfteile 15,17 mit den Rohren, sodass der zum Abdichten der Rohrverbindung nötige Anpressdruck über die Kopfteile übertragen werden kann. Die Befestigungselemente 33,35 können als Metallringe ausgebildet sein, welche am Rohr 13a resp. 13b festgelötet oder -geschweisst sind. Grundsätzlich können diese auch einstückig mit dem Rohr sein. Sie besitzen vorzugsweise Perforierungen (nicht dargestellt), welche die Verbindung zwischen Rohr und Kunststoff der als Umspritzungen der Rohrenden ausgebildeten Kopfteile verbessern.

Die Schraube 25 stösst an die betätigungsferne Stirnseite 31 des Kopfteils 17 an und ist vorzugsweise am Rohr 13b festgelötet oder angeschweisst, z.B. mittels Laserschweissen. Die Überwurfmutter 27 umschliesst die beiden Kopfteile 15,17 und ist mit der Schraube 25 verschraubt. Durch Anziehen der Überwurfmutter 27 werden die Kopfteile zusammengezogen, sodass sich eine spaltfreie Verbindung zwischen den beiden Rohrenden ergibt.

Die Überwurfmutter 27 besitzt eine Aussparung 37 für das Rohr 13a. Falls die aus den Rohren 13a,13b bestehende Leitung elektrisch leitend sein soll, kann - wie in der Figur 2 gezeigt - zwischen der Überwurfmutter 27 und dem Rohr 13a ein abgewinkeltes Kontaktblech 39 vorgesehen sein. Denkbar ist, das Kontaktblech 39 am Rohr 13a oder an der Überwurfmutter 27 festzulöten oder anzuschweissen. Auch kann das Kontaktblech 39 als Kontakthülse (nicht gezeigt) mit einer Bördelung oder endständigem Flansch ausgebildet sein, welche am Rohr 13a festgelötet oder angeschweisst ist.

Das zweite Ausführungsbeispiel gemäss Figur 3 unterscheidet sich vom Ausführungsbeispiel von Figur 1 darin, dass lediglich am Kopfteil 41 eine Dichtung 19' angeformt ist. Das mit dem Kopfteil 41 zusammenwirkende Kopfteil 43 dient deshalb ausschliesslich als Führung oder Zentrierung des Kopfteils 41.

Die Rohrverbindung ist als Umspritzung der Rohrenden realisiert. Unter Berücksichtigung des Schwundmasses des eingesetzten Kunststoffs lassen sich die Dichtungen und Führungsmittel mit grosser Genauigkeit herstellen, sodass eine exakt fluchtende, spaltfreie Verbindung zwischen zwei Metallrohren realisierbar ist. Als Kunststoff gelangt vorzugsweise ein lösungsmittelbeständiger Kunststoff, beispielsweise ein perfluorierter oder fluorhaltiger Kunststoff wie PTFE (Polytetrafluorethylen), PVDF (Poly(vinylidenfluorid), PVF (Poly(vinylfluorid), FEP (fluorhaltige Copolymere), E/TFE (Ethylen/Tetrafluorethylen-Copolymer) u.a. zum Einsatz.
Die erfindungsgemässe Rohrverbindung eignet sich für die Verbindung von Rohren mit einem Aussendurchmesser von weniger als 3 mm und vorzugsweise weniger als 2 mm Die vorliegende Erfindung beschränkt sich nicht auf die gezeigten Ausführungsbeispiele, sondern es sind verschiedene Abwandlungen denkbar. So kann die Rohrverbindung beispielsweise auch für die Verbindung von Rohren mit unterschiedlichem Durchmesser eingesetzt werden, wobei dann der Durchgang durch die Dichtungen konisch ausgebildet sein kann. Denkbar ist auch, dasselbe Verbindungsprinzip für den Anschluss eines Rohres an ein Gerät, Behälter etc. einzusetzen. Auch können die miteinander zusammenwirkenden Führungsmittel unterschiedlich ausgebildet sein, solange diese eine exakte gegenseitige Ausrichtung der beiden Rohrenden gewährleisten.

## Patentansprüche

1. Rohrverbindung (11) zur Verbindung von zwei Rohrenden (13a,13b)von zwei Rohren, mit Dichtmitteln (15,17;41) und einer Spann- oder Zusammenziehvorrichtung (23, 25) zum Zusammenziehen der Rohrenden (13a, 13b), bei welcher Rohrverbindung (11)
- auf dem ersten Rohrende (13a) ein erstes Kopfteil (15, 41) aus Kunststoff ausgebildet ist, an welchem Kopfteil (15,41) ein Flansch ausgebildet ist, welcher Flansch einen mit dem Innendurchmesser des ersten Rohrendes (13a) fluchtenden Durchgang (24) besitzt, einerseits mit der Stirnseite des ersten Rohrendes (13a) zusammenwirkt und andererseits eine sich quer zur Längsachse der Rohre erstreckende Dichtfläche (26) besitzt,
- auf dem zweiten Rohrende (13b) ein zweites Kopfteil (17) ausgebildet ist,
- und miteinander zusammenwirkende Führungsmittel (20,22) vorhanden sind, welche sicherstellen , dass bei zusammengezogenen Rohrenden (13a, 13b) der Durchgang (24) des Flansches und der Innendurchmesser des zweiten Rohrendes (13b) miteinander fluchten,
**dadurch gekennzeichnet,**
- **dass** die Rohre metallische Feinrohre sind,
- **dass** die Kopfteile (15,17;41,43) auf die Rohrenden (13a,13b) aufgespritzte Kunststoff-Umspritzungen sind,
- **dass** die miteinander zusammenwirkenden Führungsmittel (20,22) an den Kopfteilen (15,17,41,43) ausgebildet sind und die beiden Kopfteile (15,17) zusammensteckbar sind,
- und **dass** der am ersten Kopfteil (15;41) ausgebildete Flansch eine Dichtung (19) ist, deren Dichtfläche (26) bei zusammengezogenen Rohrenden (13a, 13b) spaltfrei am zweiten Rohrende (13b) oder am zweiten Kopfteil (17) anliegt.

2. Feinrohrverbindung nach Anspruch 1, **dadurch gekennzeichnet, dass** am zweiten Kopfteil (15) eine zweite Dichtung (21) angeformt ist, welche einen Durchgang (24) aufweist, der mit der Innenwandung des zweiten Rohrendes (13a,13b) fluchtet.

3. Feinrohrverbindung nach Anspruch 1 oder 2, **dadurch gekennzeichnet, dass** die Führungsmittel (20,22) als männliches und als weibliches Teil ausgebildet sind.

4. Feinrohrverbindung nach einem der Ansprüche 1 bis 3, **dadurch gekennzeichnet, dass** die Führungsmittel (20,22) der beiden Kopfteile formschlüssig miteinander zusammenwirken.

5. Feinrohrverbindung nach einem der Ansprüche 1 bis 4, **dadurch gekennzeichnet, dass** die Führungsmittel einerseits als zylindrischer Stutzen oder Zapfen (20) und andererseits als eine zylindrische Aussparung (22) ausgebildet sind.

6. Feinrohrverbindung nach Anspruch 5, **dadurch gekennzeichnet, dass** die Mantelfläche des Zapfens (20) leicht konisch ist.

7. Feinrohrverbindung nach einem der Ansprüche 1 bis 6, **dadurch gekennzeichnet, dass** in Abstand zur Stirnseite des Rohrendes (13a,13b) ein Befestigungselement (33,35) zur wenigstens axialen Fixierung des Kopfteils (15,17) vorgesehen ist.

8. Feinrohrverbindung nach einem der Ansprüche 1 bis 7, **dadurch gekennzeichnet, dass** die Kopfteile (15, 17) aus einem lösungsmittelbeständigen Kunststoff, beispielsweise einem perfluorierten oder fluorhaltigen Kunststoff wie PTFE, PVDF, PVF, FEP, E/TFE hergestellt sind,

9. Feinrohrverbindung nach Anspruch 7, **dadurch gekennzeichnet, dass** das Befestigungselement (33,35) mit Aussparungen versehen ist.

10. Feinrohrverbindung nach Anspruch 7 oder 9, **dadurch gekennzeichnet, dass** das Befestigungselement (33,35) in einem Abstand von < 3 mm, vorzugsweise < 1 mm und ganz besonders bevorzugt < 0.5 mm zur Stirnseite des Rohrendes (13a, 13b) angeordnet ist.

11. Feinrohrverbindung nach einem der Ansprüche 1 bis 10, **dadurch gekennzeichnet, dass** die Spannvorrichtung eine Schraube (25) und eine mit der Schraube zusammenwirkende, die beiden Kopfteile aufnehmende Überwurfmutter (27) gebildet ist, wobei die Überwurfmutter (27) und die Schraube (25) an den verbindungsfernen Stirnseiten (29,31) der Kopfteile (15,17) angreifen.

12. Feinrohrverbindung nach Anspruch 11, **dadurch gekennzeichnet, dass** zwischen der Schraube (25) und dem einen Rohrende (13a) und der Überwurfmutter (27) und dem anderen Rohrende (13b) eine elektrische leitende Verbindung (39) vorgesehen ist.

## Claims

1. Pipe connection (11) for connecting two pipe ends (13a, 13b) of two pipes, with sealing means (15, 17; 41) and a clamping or contracting device (23, 25) for contracting the pipe ends (13a, 13b), pipe connection (11) for which
- on the first pipe end (13a) a first head part (15, 41) made of synthetic material is configured, head part (15, 41) on which a flange is configured which possesses a passage (24) aligned with the inner diameter of the first pipe end (13a), on the one hand cooperates with the front side of the first pipe end (13a) and on the other hand possesses a sealing surface (26) extending transversely to the longitudinal axis of the pipes,
- on the second pipe end (13b) a second head part (17) is configured,
- and guiding means (20, 22) which cooperate the one with the other exist which guarantee that, the pipe ends (13a, 13b) being contracted, the passage (24) of the flange and the inner diameter of the second pipe end (13b) are aligned with each other,
**characterized in**
- **that** the pipes are metallic fine pipes,
- **that** the head parts (15, 17; 41, 43) are synthetic material coatings injection moulded onto the pipe ends (13a, 13b),
- **that** the guiding means (20, 22) which cooperate the one with the other are configured on the head parts (15, 17, 41, 43) and both head parts (15, 17) can be stuck together
- and **that** the flange configured on the first head part (15; 41) is a gasket (19), the sealing surface (26) of which rests, when the pipe ends (13a, 13b) are contracted, without any slit, on the second pipe end (13b) or on the second head part (17).

2. Fine pipe connection according to claim 1, **characterized in that** a second gasket (21) is moulded on the second head part (15), gasket which has a passage (24) which is aligned with the inner wall of the second pipe end (13a, 13b).

3. Fine pipe connection according to claim 1 or 2, **characterized in that** the guiding means (20, 22) are configured as male part and as female part.

4. Fine pipe connection according to any of the claims 1 to 3, **characterized in that** the guiding means (20, 22) of both head parts cooperate form-fittedly with each other.

5. Fine pipe connection according to any of the claims 1 to 4, **characterized in that** the guiding means are configured on the one hand as a cylindrical muff or journal (20) and on the other hand as a cylindrical recess (22).

6. Fine pipe connection according to claim 5, **characterized in that** the lateral surface of the journal (20) is slightly conical.

7. Fine pipe connection according to any of the claims 1 to 6, **characterized in that** a fixing element (33, 35) for the at least axial fixation of the head part (15, 17) is provided at a distance from the front side of the pipe end (13a, 13b).

8. Fine pipe connection according to any of the claims 1 to 7, **characterized in that** the head parts (15, 17) are produced of a solvent resistent synthetic material, for example of a perfluorated or fluoric synthetic material such as polytetrafluor ethylene, polyvinylidene fluoride, PVF, FEP, E-TFE.

9. Fine pipe connection according to claim 7, **characterized in that** the fixing element (33, 35) is provided with recesses.

10. Fine pipe connection according to claim 7 or 9, **characterized in that** the fixing element (33, 35) is placed at a distance of < 3 mm, preferably < 1 mm and particularly preferably < 0,5 mm from the front side of the pipe end (13a, 13b).

11. Fine pipe connection according to any of the claims 1 to 10, **characterized in that** the clamping device is formed by a screw (25) and an union nut (27) cooperating with the screw and receiving both head parts, whereby the union nut (27) and the screw (25) act on the connection distant front sides (29, 31) of the head parts (15, 17).

12. Fine pipe connection according to claim 11, **characterized in that** an electrically conductive connection (39) is provided between the screw (25) and the one pipe end (13a) and the union nut (27) and the other pipe end (13b).

## Revendications

1. Connexion de tuyaux (11) pour relier deux extrémités de tuyau (13a, 13b) de deux tuyaux, avec des moyens d'étanchéité (15, 17 ; 41) et un dispositif de serrage ou de contraction (23, 25) pour contracter les extrémités de tuyaux (13a, 13b), connexion de tuyaux (11) pour laquelle
- il est configuré sur la première extrémité de tuyau (13a) une première partie de tête (15, 41) en matière plastique, partie de tête (15, 41) sur laquelle une bride est configurée qui possède un passage (24) qui est aligné avec le diamètre intérieur de la première extrémité de tuyau (13a), qui coopère d'une part avec la face frontale de la première extrémité de tuyau (13a) et qui possède d'autre part une surface d'étanchéité (26) qui s'étend transversalement par rapport à l'axe longitudinal des tuyaux,
- il est configuré sur la seconde extrémité de tuyau (13b) une seconde partie de tête (17),
- et il existe des moyens de guidage (20, 22) qui coopèrent l'un avec l'autre qui assurent que, lorsque les extrémités de tuyaux (13a, 13b) sont contractées, le passage (24) de la bride et le diamètre intérieur de la seconde extrémité de tuyau (13b) sont alignées l'un avec l'autre,
**caractérisée en ce**
- **que** les tuyaux sont de fins tuyaux métalliques,
- **que** les parties de tête (15, 17 ; 41, 43) sont des gainages de matière plastique moulés par injection sur les extrémités de tuyau (13a, 13b),
- **que** les moyens de guidage (20, 22) qui coopèrent l'un avec l'autre (20, 22) sont configurés sur les parties de tête (15, 17, 41, 43) et les deux parties de têtee (15, 17) peuvent être emboîtées l'une avec l'autre
- et **que** la bride configurée sur la première partie de tête (15 ; 41) est une étanchéité (19) dont la surface d'étanchéité (26) repose, lorsque les extrémités de tuyau (13a, 13b) sont contractées, sans fente sur la seconde extrémité de tuyau (13b) ou sur la seconde partie de tête (17).

2. Connexion de tuyaux fins selon la revendication 1, **caractérisée en ce qu'**une seconde étanchéité (21) est moulée sur la seconde partie de tête (15), étanchéité qui présente un passage (24) qui est aligné avec la paroi intérieure de la seconde extrémité de tuyau (13a, 13b).

3. Connexion de tuyaux fins selon la revendication 1 ou 2, **caractérisée en ce que** les moyens de guidage (20, 22) sont configurés comme pièce mâle et comme pièce femelle.

4. Connexion de tuyaux fins selon l'une des revendications 1 à 3, **caractérisée en ce que** les moyens de guidage (20, 22) des deux parties de tête coopèrent l'une avec l'autre de manière crabotée.

5. Connexion de tuyaux fins selon l'une des revendications 1 à 4, **caractérisée en ce que** les moyens de guidage sont configurés d'une part comme tubulure cylindrique ou tourillon (20) et d'autre part comme un évidement cylindrique (22).

6. Connexion de tuyaux fins selon la revendication 5, **caractérisée en ce que** la surface de l'enveloppe du tourillon (20) est légèrement conique.

7. Connexion de tuyaux fins selon l'une des revendications 1 à 6, **caractérisée en ce qu'**un élément de fixation (33, 35) est prévu à une distance de la face frontale de l'extrémité du tuyau (13a, 13b) pour la fixation au moins axiale de la partie de tête (15, 17).

8. Connexion de tuyaux fins selon l'une des revendication 1 à 7, **caractérisée en ce que** les parties de tête (15, 17) sont fabriquées en une matière plastique résistante aux solvants, par exemple en une matière plastique perfluorée ou fluorée comme le polytétrafluoréthylène, le polyvinulidènefluorure, le polyfluorure de vinyle, le téflon, l'éthylène/le tétrafluoréthylène.

9. Connexion de tuyaux fins selon la revendication 7, **caractérisée en ce que** l'élément de fixaton (33, 35) est pourvu d'évidements.

10. Connexion de tuyaux fins selon la revendication 7 ou 9, **caractérisée en ce que** l'élément de fixation (33, 35) est placé à une distance inférieure à 3 mm, de préférence inférieure à 1 mm et de manière tout particulièrement préférée inférieure à 0,5 mm par rapport à la face frontale de l'extrémité du tuyau (13a, 13b).

11. Connexion de tuyaux fins selon l'une des revendications 1 à 10, **caractérisée en ce que** le dispositif de serrage est formé d'une vis (25) et d'un écrou-raccord (27), coopérant avec la vis, qui loge les deux parties de tête, l'écrou-raccord (27) et la vis (25) mordant sur les faces frontales (29, 31) des parties de tête (15, 17) qui sont éloignées de l'assemblage.

12. Connexion de tuyaux fins selon la revendication 11, **caractérisée en ce qu'**il est prévu une liaison électroconductrice (39) entre la vis (25) et l'une des extrémités de tuyau (13a) et l'écrou-raccord (27) et l'autre extrémité de tuyau (13b).
